# EUROPÄISCHE PATENTSCHRIFT

(11) **EP 0 421 285 B1**
(45) Veröffentlichungstag und Bekanntmachung des Hinweises auf die Patenterteilung: **30.11.1994**
(21) Anmeldenummer: 90118632.0
(22) Anmeldetag: 28.09.1990
(51) Int. Cl.: A61B 17/22, B06B 3/00

(54) **Vorrichtung zur Zertrümmerung von in Körperhöhlen befindlichen Konkrementen**
Device for the disintegration of concretions in body cavities
Appareil de destruction de concrétions dans des cavités du corps

(30) Priorität: 03.10.1989 DE 3932966
(43) Veröffentlichungstag der Anmeldung: 10.04.1991
(73) Patentinhaber: Richard Wolf GmbH, 75438 Knittlingen (DE)
(72) Erfinder: Ams, Felix, D-7539 Kämpfelbach (DE); Zanger, Ulf, D 7520 Bruchsal 4 (DE); Bolg,Ulrich, D-7519 Sulzfeld (DE)
(74) Vertreter: Wilcken, Thomas, Dipl.-Ing.

(56) Entgegenhaltungen:
- DE-A- 3 826 414
- DE-U- 8 816 114
- US-A- 4 131 505
- US-A- 4 315 181
- US-A- 4 869 715
- ULTRASONICS, Juli 1988, Seiten 198-203; D.A. HUTCHINS: "Experimental study of mode conversion at slots"

## Beschreibung

Die Erfindung betrifft eine Vorrichtung zur Zertrümmerung von in Körperhöhlen befindlichen Konkrementen gemäß dem Oberbegriff des Anspruchs 1 bzw. des Anspruchs 2.

Bei der Entfernung von Konkrementen aus dem Körperinneren von Lebewesen ist es häufig notwendig, diese Konkremente zunächst zu zerkleinern, um sie anschließend beispielsweise mit einer Saugvorrichtung einfach entfernen zu können. Für die Desintegration von Konkrementen werden häufig Vorrichtungen und Verfahren eingesetzt, welche auf der Nutzung von Ultraschallenergie beruhen. Hierbei kann die Zerkleinerung oder Desintegration des Konkrementes mit berührungsfrei oder aber auch unter Berührung des Konkrementes arbeitenden Instrumenten und Geräten erfolgen.

Die unter Berührung des Konkrementes arbeitenden Instrumente sind üblicherweise mit einer stab- oder röhrenförmig ausgebildeten Sonotrode ausgestattet, die an das zu zerstörende Konkrement herangeführt und durch einen Ultraschallwandler in Schwingungen versetzt wird. Aufgrund des stab- oder röhrenförmigen Aufbaus der Sonotrode erfolgt die Zerkleinerung der Konkremente im Körper im wesentlichen dadurch, daß mechanische longitudinale Schwingungen auf das Konkrement übertragen werden. Hieraus resultiert häufig lediglich ein Durchbohren des Konkrementes, so daß die Sonotrode zur vollständigen Zerstörung des Konkrementes mehrmalig an verschiedenen Stellen des Konkrementes angesetzt werden muß. Ziel einer Therapie ist es aber stets, eine hinreichende Zerkleinerung des Konkrementes innerhalb kürzester Zeit herbeizuführen, um die Belastung des Patienten so gering als möglich zu halten.

Aus der DE-PS 22 19 790 ist ein rohrförmiger Ultraschallschwinger bekannt, der an seinem distalen Ende mit einem mit verschränkten Zähnen versehenen, lose gekoppelten Prallkörper ausgestattet ist. Mit diesem soll es möglich sein, auch harte Körpersteine durch Sprödbrüche zum Zerplatzen zu bringen. Als nachteilig hat es sich bei diesem Vorschlag erwiesen, daß der Zeitraum bis zu dem Zerplatzen des Steines noch immer zu lang ist. Dies ist darauf zurückzuführen,daß praktisch nur longitudinale Schwingungen für die Desintegration des Konkrementes herangezogen werden.

Eine Vorrichtung zum Zerkleinern von Konkrementen im Harnleiter in Anwendung einer komplexen Einwirkung durch Ultraschallvibration und elektrohydraulische Stöße auf das Konkrement ist in der DE-OS 27 33 019 beschrieben. Bei dieser Vorrichtung besteht die Sonotrode aus einem Draht, dessen distales Ende mit Zähnen versehen ist. Zusätzlich kann dieser Draht an seinem distalen Ende mit längsverlaufenden Vorsprüngen und Nuten versehen sein. Zur Einwirkung der Ultraschallvibration auf das Konkrement wird das distale Ende der Sonotrode mittels eines Ultraschallwandlers in Längs- und Querschwingungen versetzt. Unter einer regelrechten Fräswirkung wird das Konkrement so zerkleinert. Hernach wird die Sonotrode gegen eine Sonde ausgetauscht, mit welcher nach dem Prinzip der elektrohydraulischen Lithotripsie das Konkrement weiter zerkleinert wird.Als Nachteil dieser Vorrichtung wird es empfunden, daß ihre Anwendung recht umständlich und daher zeitintensiv ist.Zudem birgt die elektrohydraulische Lithotripsie die Gefahr in sich, daß beim Zerplatzen des Konkrements das umgebende Gewebe durch Fragmente verletzt wird, woraus sich Folgekrankheiten entwickeln können.

Eine weitere Vorrichtung zur Harnsteinzertrümmerung ist in der US-PS 38 30 240 beschrieben.Darin wird vorgeschlagen, die Steinzertrümmerung mit einer ebenfalls aus Draht gebildeten Sonotrode vorzunehmen, welche sowohl am Verbindungsstück zum Wandler als auch an ihrem distalen Ende unterschiedliche Formen aufweisen kann. Hierdurch sollen am distalen Ende der Sonotrode Längs- und Querschwingungen erzeugt werden können. Aufgrund der Formgebung der Drahtenden sowohl am distalen, als auch am proximalen Verbindungsbereich zum Wandler ergibt sich der schwerwiegende Nachteil, daß diese Drahtsonotroden im Betrieb sehr leicht brechen können und zu Verletzungen des umgebenden Gewebes führen können.

Schließlich ist in der DE-A-38 26 414 noch ein weiteres Ultraschall-Therapiegerät für die Zerstörung von Konkrementen in Patienten beschrieben. Es besteht aus einer distal vorgesehenen Sonde für die Vibrationsübertragung, einem in seiner äußeren Form symmetrischen Horn in annähernd kegelstumpfförmiger Gestalt und einem proximalen Reflektor, der eine unsymmetrische äußere Formgestalt aufweisen kann. Der Reflektor ist mittels einer exzentrisch angeordneten Mutter-Bolzen-Verbindung rückwärtig an dem Horn angeschraubt.

Vor dem aufgezeigten Hintergrund ist es nun die Aufgabe der vorliegenden Erfindung, eine eingangs erwähnte Vorrichtung so weiterzubilden, daß die Zerstörung von Konkrementen in kürzester Zeit unter Vermeidung der erwähnten Nachteile bekannter Vorrichtungen ermöglicht wird.

Gelöst wird diese Aufgabe durch die Vorrichtung gemäß dem Anspruch 1 oder 2.

Demgemäß ist das Horn der Vorrichtung nach der ersten Ausführungsform mit Vertiefungen auf seiner Oberfläche versehen, die nicht parallel zu seiner Symmetrieachse verlaufen. Hierdurch werden neben longitudinalen Schwingungen Quer- und Rotationsschwingungen erzeugt, die eine sehr schnelle Zerkleinerung des zu zerstörenden Konkrementes ermöglichen.

Dieser Effekt kann dadurch erhöht werden, daß die Sonotrode an ihrem distalen Ende mit Schlitzen versehen ist, die nicht parallel zu der Symmetrieachse der Sonotrode verlaufen und deren Länge in etwa λ/4 beträgt, wobei λ der Wellenlänge der erzeugten Querschwingungen entspricht.

Diese Ausführungsform kann in vorteilhafter Weise noch dadurch weitergebildet werden, daß die zwischen zwei benachbarten Schlitzen begrenzten Lamellen zusätzlich mit Einkerbungen versehen sind.

Wenn das Horn und/oder der Reflektor der Vorrichtung nach der zweiten Ausführungsform eine unsymmetrische Form aufweisen, wobei beim Horn die äußere Formgestalt unsymmetrisch ist, lassen sich weitere Quer- und Rotationsschwingungen erzeugen, die zu einer schnellen Zerkleinerung des Konkrementes beitragen. Die unsymmetrische Form läßt sich beispielsweise durch einseitige flächenhafte Ausfräsungen erzeugen.

Um die durch die Formgebung des Horns und/oder Reflektors erzeugten Quer- und Rotationsschwingungen ebenso wie die erzeugten Longitudinalschwingungen optimal an die Sonotrodenspitze zu leiten, ist es vorteilhaft, wenn die Sonotrode und das Horn aus Werkstoffen mit im wesentlichen gleicher akustischer Impedanz bestehen. Als besonders geeignet hat sich Titan als Material für das Horn und die Sonotrode erwiesen. Ähnlich günstig für eine optimale Energieübertragung ist ein Horn aus Duraluminium und eine Sonotrode aus Titan.

Die Erfindung wird anhand eines Ausführungsbeispiels gemäß der Zeichnungen erläutert. Hierbei zeigt:
- Fig. 1: eine Ausführungsform der erfindungsgemäßen Vorrichtung mit dem zugehörigen Ultraschallgenerator, und
- Fig. 2: die vergrößerte Seitenansicht des distalen Endes der Sonotrode der Vorrichtung gemäß Fig. 1.

Die erfindungsgemäße Vorrichtung besteht im wesentlichen aus mindestens einem an einen Ultraschallgenerator 1 anschließbaren piezoelektrischen Wandler, der vorliegend als aus zwei piezokeramischen Scheiben 10 bestehend dargestellt ist. Diese piezokeramischen Scheiben sind eingefaßt von einem Reflektor 8 und von dem Horn 9. Mit dem Horn 9 ist lösbar verbunden die Sonotrode 3, durch die die vom Wandler erzeugten Ultraschallwellen zu einem zu zerstörenden Konkrement 11 geleitet werden.

Der Ultraschallgenerator 1 ist von bekannter Bauart, weswegen hier nur kurz auf seine Funktion eingegangen wird. Er enthält zur Erzeugung einer elektrischen Schwingung im wesentlichen einen spannungsgesteuerten Oszillator 4, dessen Ausgangssignal über einen Ausgangsverstärker 5 und einen Ausgangsübertrager 6 an den Ultraschallwandler 2 gegeben wird. Dabei wird von einem Phasenvergleicher 7, der die Phasen der Ausgangsspannung und des Ausgangsstroms des Ausgangsübertragers 6 vergleicht, eine Regelspannung zur Steuerung des Oszillators 4 erzeugt und diesem zugeführt.

Das im Ultraschallgenerator 1 erzeugte elektrische Signal wird dem Ultraschallwandler 2 zugeführt und von diesem in mechanische Schwingungen umgewandelt. Die Sonotrode 3 ist vorliegend rohrförmig ausgebildet, so daß durch ihren Hohlraum, der bis zu ihrem proximalen Ende reicht und durch den Ultraschallwandler 2 hindurch bis zu einem Sauganschluß 17 weitergeführt ist, die Absaugung der Steinfragmente und ggf. der Spülflüssigkeit vorgenommen werden kann.

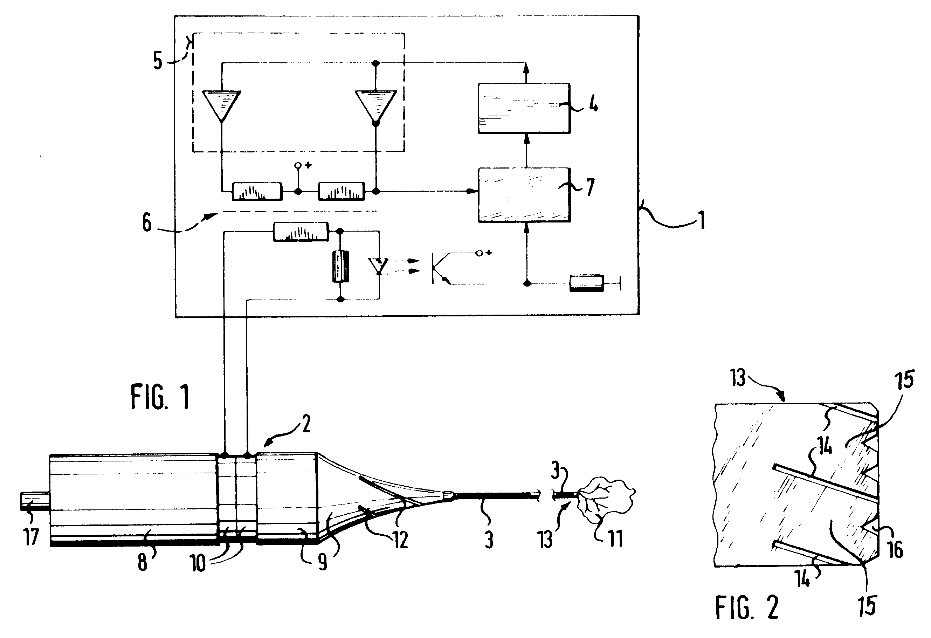

Zur Erzeugung eines hohen Anteils an Rotations- und Querschwingungen sind am Horn 9 des Ultraschallwandlers 2 Vertiefungen 12 so eingefräst, daß sie nicht parallel zur Symmetrieachse des Horns 9 verlaufen. Dem gleichen Zweck dienen in dem in Fig. 2 vergrößert dargestellten distalen Ende 13 der Sonotrode 3 eingebrachte Schlitze 14.Diese sind so angeordnet, daß sie nicht parallel zu der Symmetrieachse der Sonotrode verlaufen. Ihre Länge beträgt in etwa einem Viertel der Wellenlänge der erzeugten Querschwingungen.Die Schlitze 14 sind so schmal ausgeführt, daß eine hinreichende Absaugwirkung durch die Sonotrode 3 hindurch erhalten bleibt.

Die zwischen zwei benachbarten Schlitzen 14 ausgebildeten Lamellen 15 sind zusätzlich mit Zähnen oder Einkerbungen 16 versehen, die eine zusätzliche Fräswirkung der Sonotrodenspitze hervorrufen.

Zur zusätzlichen Erzeugung von Quer- und Rotationsschwingungen können das Horn 9 und/oder der Reflektor unsymmetrisch geformt sein. Dies kann beispielsweise durch einseitige flächenhafte Ausfräsungen erzielt werden, die vorliegend nicht dargestellt sind.

Eine optimale Energieübertragung zwischen dem Ultraschallwandler 2 und der Sonotrode 3 zum Konkrement 11 hin wird insbesondere dadurch erreicht, daß die Sonotrode 3 und das Horn 9 des Ultraschallwandlers 2 aus dem gleichen Werkstoff, zumindest aber aus Werkstoffen mit im wesentlichen gleicher akustischer Impedanz bestehen. So kann das Horn 9 beispielsweise aus Duraluminium oder Titan und die Sonotrode 3 aus Titan gefertigt sein. Durch die Verwendung von Titan anstelle des üblicherweise eingesetzten Edelstahls als Werkstoff für die Sonotrode 3 sind höhere Schwingungsamplituden und damit geringere Zeiten zur Zertrümmerung des Konkrementes erreichbar.Gleichzeitig wird bei Einsatz dieses Werkstoffes eine erhebliche Reduzierung der Wärmeverluste erzielt.

## Patentansprüche

1. Vorrichtung zur Zertrümmerung von in Körperhöhlen befindlichen Konkrementen, bestehend aus einem Ultraschallwandler (2) zwischen einem Reflektor (8) und einem Horn (9), von dem aus mittels einer Sonotrode (3) die erzeugten Ultraschallwellen an die Konkremente (11) geleitet werden, dadurch gekennzeichnet, daß das Horn (9) mit zu seiner Symmetrieachse unparallel verlaufenden Vertiefungen (12) auf seiner Oberfläche versehen ist.

2. Vorrichtung zur Zertrümmerung von in Körperhöhlen befindlichen Konkrementen, bestehend aus einem Ultraschallwandler (2) zwischen einem Reflektor (8) und einem Horn (9), von dem aus mittels einer Sonotrode (3) die erzeugten Ultraschallwellen an die Konkremente (11) geleitet werden, wobei der Reflektor eine unsymmetrische äußere Formgestalt aufweisen kann, dadurch gekennzeichnet, daß das Horn (9) in seiner äußeren Formgestalt unsymmetrisch ausgebildet und mit Vertiefungen (12) auf seiner Oberfläche versehen ist.

3. Vorrichtung nach Anspruch 1 oder 2, dadurch gekennzeichnet, daß die Sonotrode (3) an ihrem distalen Ende (13) mit zu ihrer Symmetrieachse unparallel verlaufenden Schlitzen (14) versehen ist, deren Länge in etwa λ/4 beträgt, wobei λ der Wellenlänge der erzeugten Querschwingungen entspricht.

4. Vorrichtung nach Anspruch 3, dadurch gekennzeichnet, daß die von zwei benachbarten Schlitzen (14) begrenzten Lamellen (15) zusätzlich mit Einkerbungen (16) versehen sind.

5. Vorrichtung nach Anspruch 1 oder 2, dadurch gekennzeichnet, daß die Sonotrode (3) und das Horn (9) aus Werkstoffen mit im wesentlichen gleicher akustischer Impedanz bestehen.

6. Vorrichtung nach Anspruch 5, dadurch gekennzeichnet, daß das Horn (9) und die Sonotrode (3) aus Titan bestehen.

7. Vorrichtung nach Anspuch 5, dadurch gekennzeichnet, daß das Horn (9) aus Duraluminium und die Sonotrode (3) aus Titan besteht.

## Claims

1. A device for the disintegrating of concretions situated in body cavities, consisting of an ultrasonic transducer (2) between a reflector (8) and a horn (9), from which, by means of a sonotrode (3), the produced ultrasonic waves are directed against the concretions (11), characterised in that the horn (9) is provided on its surface with depressions (12) running in a non-parallel manner to its axis of symmetry.

2. A device for the disintegrating of concretions situated in body cavities, consisting of an ultrasonic transducer (2) between a reflector (8) and a horn (9), from which, by means of a sonotrode (3), the produced ultrasonic waves are directed against the concretions (11), in which the reflector can have an asymmetrical external shape, characterised in that the horn (9) is constructed so as to be asymmetrical in its external shape and is provided with depressions (12) on its surface.

3. A device according to Claim 1 or 2, characterised in that the sonotrode (3) is provided on its distal end (13) with slits (14) running in a non-parallel manner to its axis of symmetry, the length of which slits amounts to approximately λ/4, in which λ corresponds to the wave length of the produced transverse oscillations.

4. A device according to Claim 3, characterised in that the sheets (15) delimited by two adjacent slits (14) are additionally provided with notches (16).

5. A device according to Claim 1 or 2, characterised in that the sonotrode (3) and the horn (9) consist of materials with substantially identical acoustic impedance.

6. A device according to Claim 5, characterised in that the horn (9) and the sonotrode (3) consist of titanium.

7. A device according to Claim 5, characterised in that the horn (9) consists of duralumin and the sonotrode (3) consists of titanium.

## Revendications

1. Dispositif destiné à détruire des concrétions se trouvant dans des cavités du corps, composé d'un transducteur à ultrasons (2) entre un réflecteur (8) et une corne (9), à partir de laquelle les ondes ultrasonores produites, sont conduites aux concrétions (11), au moyen d'une sonotrode (3), caractérisé en ce que la corne (9) est pourvue sur sa surface, d'encoches (12) s'étendant de manière non parallèle à son axe de symétrie.

2. Dispositif destiné à détruire des concrétions se trouvant dans des cavités du corps, composé d'un transducteur à ultrasons (2) entre un réflecteur (8) et une corne (9), à partir de laquelle les ondes ultrasonores produites, sont conduites aux concrétions (11), au moyen d'une sonotrode (3), le réflecteur pouvant présenter une forme extérieure de configuration non symétrique, caractérisé en ce que la corne (9) est d'une configuration non symétrique quant à sa forme extérieure, et est pourvue d'encoches (12), sur sa surface.

3. Dispositif selon la revendication 1 ou 2, caractérisé en ce que la sonotrode (3) est pourvue, à son extrémité distale (13), de fentes (14) s'étendant de manière non parallèle par rapport à leur axe de symétrie, et dont la longueur vaut environ λ/4, λ correspondant à la longueur d'onde des vibrations transversales engendrées.

4. Dispositif selon la revendication 3, caractérisé en ce que les lamelles (15) limitées par deux fentes (14) voisines, sont en outre pourvues d'entailles (16).

5. Dispositif selon la revendication 1 ou 2, caractérisé en ce que la sonotrode (3) et la corne (9) sont réalisées en des matériaux présentant sensiblement la même impédance acoustique.

6. Dispositif selon la revendication 5, caractérisé en ce que la corne (9) et la sonotrode (3) sont réalisées en titane.

7. Dispositif selon la revendication 5, caractérisé en ce que la corne (9) est réalisée en duralumin, et la sonotrode (3) en titane.
